(19) **Europäisches Patentamt European Patent Office Office européen des brevets**

(11) **EP 4 675 641 A1**

(12) **EUROPEAN PATENT APPLICATION**
published in accordance with Art. 153(4) EPC

(43) Date of publication:
07.01.2026 Bulletin 2026/02

(21) Application number: 23924555.8

(22) Date of filing: 28.02.2023

(51) International Patent Classification (IPC):
$G16H\ 50/30^{(2018.01)}$ $A61M\ 5/172^{(2006.01)}$

(52) Cooperative Patent Classification (CPC):
A61M 5/172; G16H 50/30

(86) International application number:
PCT/CN2023/078582

(87) International publication number:
WO 2024/178575 (06.09.2024 Gazette 2024/36)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(71) Applicant: **Medtrum Technologies Inc.**
Shanghai 201203 (CN)

(72) Inventor: **YANG, Cuijun**
Shanghai 201203 (CN)

(74) Representative: **Vogelbruch, Keang**
**VOGELBRUCH Patentanwaltsgesellschaft mbH**
**Paul-Gerhardt-Straße 16**
**40593 Düsseldorf (DE)**

(54) **AUTOMATIC MONITORING METHOD BASED ON RATE OF CHANGE OF DIFFERENCE BETWEEN ACTUAL BLOOD GLUCOSE VALUES, AND CLOSED-LOOP ARTIFICIAL PANCREAS**

(57) The invention discloses an automatic detection method based on the change rate of the difference between the actual blood glucose values, including: obtaining an actual blood glucose value of the user at the current time; obtaining a historical actual blood glucose value of the user at the previous time, and calculate a difference between the actual blood glucose values at the current time and at the previous time; calculating a change rate of the difference between the actual blood glucose values at the current time; and comparing the change rate of the difference between the actual blood glucose values at the current time with a preset threshold, and determining an event type according to the comparing result. According to the determined event type, the artificial pancreas can automatically adjust the corresponding infusion strategy to achieve closed-loop control of the artificial pancreas.

FIG.2

## Description

### TECHNICAL FIELD

The present invention mainly relates to the field of medical device, and in particular, to a method for automatic detecting meal and/or exercise events and the closed-loop artificial pancreas thereof.

### BACKGROUND

The pancreas of healthy people can automatically secrete the required insulin/glucagon according to the glucose level in the human blood, thereby maintaining a reasonable range of blood glucose fluctuations. However, for diabetic patients, the function of their pancreas has been severely compromised, and the pancreas cannot secrete the required dosage of insulin. Therefore, diabetes mellitus is defined as a metabolic disease caused by abnormal pancreatic function, and it is also classified as one of the top three chronic conditions by the WHO. The present medical advancement has not been able to find a cure for diabetes mellitus. Yet, the best the technology could do is control the onset symptoms and complications by stabilizing the blood glucose level for diabetes patients.

Diabetic patients on an insulin pump need to check their blood glucose before infusing insulin into their bodies. At present, most detection methods can continuously detect blood glucose and send the blood glucose data to the remote device in real-time for the user to view. This detection method is called Continuous Glucose Monitoring (CGM), which requires the detection device to be attached to the surface of the patients' skin, and the sensor carried by the device to be inserted into the interstitial fluid for testing. According to the blood glucose (BG) level, the infusion system mimics an artificial pancreas to fill the gaps of the required insulin amount via the closed-loop pathway or the semi-closed-loop pathway.

At present, the artificial pancreas still requires manual input of meal and/or exercise information, including meal type and size, exercise type and intensity, before the artificial pancreas adjust the corresponding infusion strategy, therefore, the current artificial pancreas can only be called a semi-closed-loop artificial pancreas. To achieve closed-loop control of the artificial pancreas, automatic meal detection and/or exercise detection of the artificial pancreas must be realized first, and then automatically adjust the corresponding infusion strategy based on the automatically detected meal and/or exercise information.

Therefore, in the prior art, there is an urgent need for a method and a closed-loop artificial pancreas that can automatically perform meal detection and/or exercise detection.

### BRIEF SUMMARY OF THE INVENTION

The embodiment of the present invention discloses an automatic detection method based on the change rate of the difference between the actual blood glucose values, including: obtaining an actual blood glucose value of the user at the current time; obtaining a historical actual blood glucose value of the user at the previous time, and calculate a difference between the actual blood glucose values at the current time and at the previous time; calculating a change rate of the difference between the actual blood glucose values at the current time; and comparing the change rate of the difference between the actual blood glucose value at the current time with a preset threshold, and determining an event type according to the comparing result. According to the determined event type, the artificial pancreas can automatically adjust the corresponding infusion strategy to achieve closed-loop control of the artificial pancreas.

The invention discloses an automatic detection method based on the change rate of the difference between the actual blood glucose values, comprising: obtaining an actual blood glucose value of the user at the current time; obtaining a historical actual blood glucose value of the user at the previous time, and calculate a difference between the actual blood glucose values at the current time and at the previous time; calculating a change rate of the difference between the actual blood glucose values at the current time; and comparing the change rate of the difference between the actual blood glucose value at the current time with a preset threshold, and determining an event type according to the comparing result.

According to one aspect of the present invention, when the change rate of the difference between the actual blood glucose values at the current time is positive and greater than a preset positive threshold, the event is determined as eating.

According to one aspect of the present invention, when the change rate of the difference between the actual blood glucose values at the current time is negative and less than a preset negative threshold, the event is determined as exercise.

According to one aspect of the present invention, when the event type is initially determined, the time interval between the current moment and the previous moment is gradually shortened.

According to one aspect of the present invention, further determine the event type in combination with other judgment results.

According to one aspect of the present invention, the other judgment results are based on the change rate of the difference between the actual blood glucose value and the predicted blood glucose value at the current time.

According to one aspect of the present invention, when the judgment result obtained from the change rate of the difference between the actual blood glucose value and the predicted blood glucose value at the current time is consistent with the judgment result obtained from the change rate of the difference between the actual blood glucose values, the event type is determined.

According to one aspect of the present invention, the influence of insulin on board and insulin sensitivity are considered in the prediction algorithm for predicting blood glucose value.

According to one aspect of the present invention, the predicted blood glucose value is at least calculated based on the actual blood glucose value at the previous moment and the first or second derivative of the actual blood glucose value at the previous moment relative to time.

According to one aspect of the present invention, the other judgment results further include the results determined according to the insulin infusion information.

According to one aspect of the present invention, the method for determining the result according to the insulin infusion information includes: obtaining an insulin infusion information in a recent time period and calculating an insulin infusion amount $I_1$; obtaining an insulin infusion information in a past time period of time and calculating an insulin infusion amount $I_2$; calculating the absolute value of the difference between $I_1$ and $I_2$; comparing an absolute value of the difference with a preset threshold value, if the absolute value of the difference is not greater than the preset threshold value, trust the result determined by the change rate of the difference between the actual blood glucose values and/or the change rate of the difference between the actual blood glucose value and the predicted blood glucose value at the current time.

According to one aspect of the present invention, the past time period is adjacent to the latest time period.

According to one aspect of the present invention, the other judgment results further include the results determined according to a variance between the actual blood glucose value and the predicted blood glucose value at the current time.

According to one aspect of the present invention, the method for determining the result based on the variance between the actual blood glucose value and the predicted blood glucose value at the current time includes: comparing the variance with a preset threshold, if the variance is greater than the threshold, trust the result determined based on the change rate of the difference between the actual blood glucose values and/or the change rate of the difference between the actual blood glucose value and the predicted blood glucose value.

According to one aspect of the present invention, the other judgment results further include the results determined based on insulin infusion information.

According to one aspect of the present invention, when the absolute value of the difference between the insulin infusion amount $I_1$ in the recent time period and the insulin infusion amount $I_2$ in the past time period is less than a preset threshold, trust the result determined according to the variance between the actual blood glucose value and the predicted blood glucose value at the current time.

The invention discloses a closed-loop artificial pancreas, including: a detection module, for continuously detecting a real-time blood glucose value of a user; an infusion module, for performing currently needed insulin infusion to the user according to an insulin infusion instruction; and an electronic module, for controlling the operation of the detection module and the infusion module, including a memory and a processor, wherein, the processor automatically determines an event type according to a change rate of the difference between actual blood glucose values calculated based on the actual blood glucose values detected by the detection module.

According to one aspect of the present invention, the method for automatically determining the type of event includes: obtaining an actual blood glucose value of the user at the current time; obtaining a historical actual blood glucose value of the user at the previous time, and calculate a difference between the actual blood glucose values at the current time and at the previous time; calculating a change rate of the difference between the actual blood glucose values at the current time; and comparing the change rate of the difference between the actual blood glucose value at the current time with a preset threshold, and determining event type according to the comparing result.

According to one aspect of the present invention, when the change rate of the difference between the actual blood glucose values at the current time is positive and greater than a preset positive threshold, the event is determined as eating; when the change rate of the difference between the actual blood glucose values at the current time is negative and less than a preset negative threshold, the event is determined as exercise.

According to one aspect of the present invention, the method of automatically determining the event type is further combined with other judgment results to jointly determine the type of event.

According to one aspect of the present invention, the other judgment results are based on the change rate of the difference between the actual blood glucose value and the predicted blood glucose value at the current time.

According to one aspect of the present invention, the other judgment results further include the results determined according to the insulin infusion information.

According to one aspect of the present invention, the method for determining the result according to the insulin infusion information includes: obtaining an insulin infusion information in the recent time period and calculating the insulin infusion amount $I_1$; obtaining an insulin infusion information in the past time period of time and calculating the insulin infusion amount $I_2$; calculating the absolute value of the difference between $I_1$ and $I_2$; comparing an absolute value of the difference with a preset threshold value, if the absolute value of the difference is not greater than the preset threshold value, trust the result determined by the change rate of the difference between the actual blood glucose values and/or the change rate of the difference between the actual blood glucose value and the predicted blood glucose value at the current time.

According to one aspect of the present invention, the other judgment results further include the results determined according to a variance between the actual blood glucose value and the predicted blood glucose value at the current time.

According to one aspect of the present invention, the method for determining the result based on the variance between the actual blood glucose value and the predicted blood glucose value at the current time includes: comparing the variance with a preset threshold, if the variance is greater than the threshold, trust the result determined based on the change rate of the difference between the actual blood glucose values and/or the change rate of the difference between the actual blood glucose value and the predicted blood glucose value.

According to one aspect of the present invention, the results determined according to the variance between the actual blood glucose value and the predicted blood glucose value at the current time further include the results determined according to the insulin infusion information.

According to one aspect of the present invention, further includes a motion sensor, which is a combination of a three-axis acceleration sensor and a gyroscope.

According to one aspect of the present invention, the detection module, the infusion module and the electronic module are at least connected or integrated to form a whole part.

According to one aspect of the present invention, the detection module, the infusion module and the electronic module are arranged in different parts, respectively.

Compared with the prior art, the technical solution of the present invention has the following advantages:

In the automatic detection method based on the change rate of the difference between the actual blood glucose values disclosed in the present invention, comparing the change rate of the difference between the actual blood glucose value at the current time with a preset threshold, and determining an event type according to the comparing result. when the change rate of the difference between the actual blood glucose values at the current time is positive and greater than a preset positive threshold, the event is determined as eating; when the change rate of the difference between the actual blood glucose values at the current time is positive and greater than a preset positive threshold, the event is determined as eating. According to the determined event type, the artificial pancreas can automatically adjust the corresponding infusion strategy to achieve closed-loop control of the artificial pancreas.

Furthermore, the automatic detection method based on the change rate of the difference between the actual blood glucose value can be combined with other judgment results to determine the event type to make the judgment results more accurate, such as combining the judgment results based on the change rate of the difference between the actual blood glucose value and the predicted blood glucose value at the current time, combining the results determined based on the insulin infusion information, and/or combining the results determined based on the difference between the actual blood glucose value and the predicted blood glucose value at the current time.

Furthermore, when the meal or exercise is detected, the detection module can shorten the detection interval, for example, Ts changes from 2 minutes to 1 minute, half a minute, 20 seconds, 10 seconds, 5 seconds, or even shorter, and increase the sampling frequency can identify the meal or exercise event more accurately.

**BRIEF DESCRIPTION OF THE DRAWINGS**

FIG.1 is a schematic diagram of the module relationship of the closed-loop artificial pancreas insulin infusion control system according to one embodiment of the present invention.

FIG.2 is a flow chart of determining the meal or exercise event based on the rate of change of the difference in the actual blood glucose value according to one embodiment of the present invention.

FIG.3 is a flow chart of determining the meal or exercise event based on the rate of change of the difference between the actual blood glucose value and the predicted blood glucose value according to one embodiment of the present invention.

FIG.4 is a flow chart of determining the meal or exercise event by combining the insulin infusion information according to one embodiment of the present invention.

FIG.5 is a flow chart of determining the meal or exercise event based on variance of actual blood glucose value and predicted blood glucose value according to one embodiment of the present invention.

## DETAILED DESCRIPTION

As mentioned above, the present artificial pancreas still requires manual input of meal and/or exercise information, including meal type and size, exercise type and intensity, before the artificial pancreas adjust the corresponding infusion strategy, therefore, only a semi-closed-loop control of the current artificial pancreas can be realized.

In order to solve this problem, the present invention provides an automatic detection method based on the change rate of the difference between the actual blood glucose values, including: obtaining an actual blood glucose value of the user at the current time; obtaining a historical actual blood glucose value of the user at the previous time, and calculate a difference between the actual blood glucose values at the current time and at the previous time; calculating a change rate of the difference between the actual blood glucose values at the current time; and comparing the change rate of the difference between the actual blood glucose values at the current time with a preset threshold, and determining an event type according to the comparing result. According to the determined event type, the artificial pancreas can automatically adjust the corresponding infusion strategy to achieve closed-loop control of the artificial pancreas.

Various exemplary embodiments of the present invention will now be described in detail with reference to the drawings. The relative arrangement of the components and the steps, numerical expressions and numerical values set forth in the embodiments are not to be construed as limiting the scope of the invention.

In addition, it should be understood that, for ease of description, the dimensions of the various components shown in the figures are not necessarily drawn in the actual scale relationship, for example, the thickness, width, length or distance of certain units may be exaggerated relative to other parts.

The following description of the exemplary embodiments is merely illustrative, and is not intended to be in any way limiting the invention and its application or use. The techniques, methods, and devices that are known to those of ordinary skill in the art may not be discussed in detail, but such techniques, methods, and devices should be considered as part of the specification.

It should be noted that similar reference numerals and letters indicate similar items in the following figures. Therefore, once an item is defined or illustrated in a drawing, it will not be discussed further in the following description of the drawings.

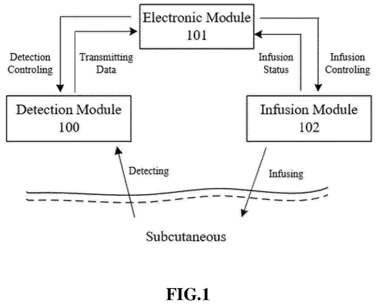

FIG.1 is a schematic diagram of the module relationship of the closed-loop artificial pancreas insulin infusion control system according to the embodiment of the present invention.

The closed-loop artificial pancreas insulin infusion control system disclosed in the embodiment of the present invention mainly includes a detection module 100, an electronic module 101, and an infusion module 102.

The detection module 100 is used to continuously detect the user's real-time blood glucose (BG) level. Generally, detection module 100 is a Continuous Glucose Monitoring (CGM) for detecting real-time BG, monitoring BG changes, and sending them to the electronic module 101 and/or the infusion module 102. the detection module 100 also includes a communication interface to communicate with external devices.

The infusion module 102 includes the essential mechanical assemblies used to infuse insulin, such as, a reservoir for storing medication; a medication infusion path including an infusion needle for infusing medication to a user; a drive component for transferring medication from the reservoir to the user via the medication infusion path; a battery for providing electrical power, etc. The infusion module 102 is provided with a program unit, including memory and processor, etc., and also includes a communication interface for communication with external devices. The infusion module 102 can infuse the currently required insulin into the user according to the insulin infusion instructions. At the same time, the infusion status of the infusion module 102 can be fed back to other modules, such as the electronic module 101, in real time. The infusion module 102 can be a conventional insulin pump or a tubeless patch insulin pump of Medtrum.

Electronic module 101 can control the detection module 100 and the infusion module 102. The electronic module 101 is a portable electronic device, such as a smart phone, PDM, smart watch, handset, etc. The portable electronic device can include a communication interface for communicating with the detection device, infusion device and external remote devices; a display and a display controller, which can present visual information and control the presentation of visual information in graphics and/or text; input devices, such as mouse, keyboard, touch screen, microphone, etc., are used to receive signal input; memory and processor, etc. the memory is used to store data, record, instructions, or store one or more control application programs for the processor to execute, etc., the processor is used to execute the instructions in the memory, calculate the insulin infusion data currently required by the user, control the operation of other components, etc.

In another embodiment of the invention, the electronic module 101 can also be a structure electrically connected or integrated with the infusion module 102 and/or the detection module 100, but has the same functions as the aforementioned portable electronic device.

The communication mode between detection module 100, infusion module 102 and electronic module 101 includes, but is not limited to, any wired or wireless communication link operated according to any known communication protocol or standard, such as Bluetooth ®, Wi-Fi, near-field communication standard, cellular standard or any other wireless protocol.

The embodiment of the present invention does not limit the specific positions and connection relationships of the detection module 100, the electronic module 101 and the infusion module 102, as long as the aforementioned functional conditions can be satisfied.

As in an embodiment of the present invention, the three are electrically connected or integrated to form a single part. Therefore, the three modules can be attached on only one position of the user's skin. If the three modules are connected as a whole and attached in only one position, the number of the device on the user skin will be reduced, thereby reducing the interference of more attached devices on user activities. At the same time, it also effectively solves the problem of poor wireless communication between separating devices, further enhancing the user experience. Generally, the service life of detection module 100, electronic module 101 and infusion module 102 is different. Therefore, when the three are electrically connected to form one equipment, they can also be separated from each other. If a module terminates its life first, the user can only replace the module and keep the other two modules for further use.

Another embodiment of the present invention is that the electronic module 101 and the infusion module 102 are electrically connected or integrated to form a single part, while the detection module 100 is separately provided in another part. At this time, the detection module 100 and the electronic module 101 transmit wireless signals to realize the mutual connection. Therefore, electronic module 101 and infusion module 102 can be attached to the user's skin position while the detection module 100 is attached to the other position.

Another embodiment of the present invention is that the electronic module 101 and the detection module 100 are electrically connected or integrated, forming a single part, while the infusion module 102 is separately provided in another part. The infusion module 102 and the electronic module 101 transmit wireless signals to realize the mutual connection. Therefore, electronic module 101 and the detection module 100 can be attached to the same position of the user's skin while the infusion module 102 is attached to the other position.

Another embodiment of the present invention is that the infusion module 102 and the detection module 100 are electrically connected or integrated, forming a single part, while the electronic module 101 is separately provided in another part. The infusion module 102, the detection module 100 and the electronic module 101 transmit wireless signals to realize the mutual connection. Therefore, the infusion module 102 and the detection module 100 can be attached to the same position of the user's skin while the electronic module 101 is attached to the other position, or carry on as a portable electronic device.

Another embodiment of the present invention is that the three are provided in different parts, thus being attached to different positions, or the infusion module 102 and the detection module 100 being attached to different positions, and the electronic module 101 being carried on as a portable electronic device. Simultaneously, electronic module 101, detection module 100, and infusion module 102 transmit wireless signals to realize the mutual connection.

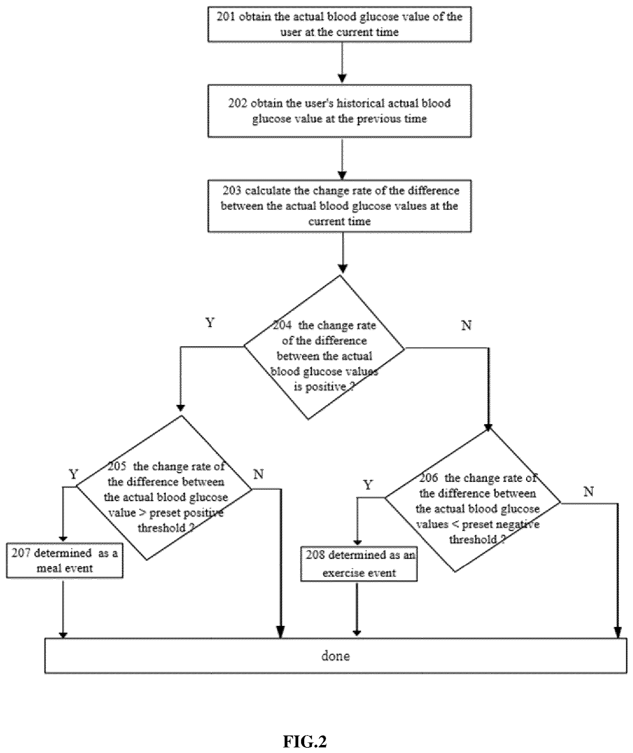

FIG.2 is a flow chart of determining the meal or exercise event based on the rate of change of the difference in the actual blood glucose value according to one embodiment of the present invention.

The detection module 100 detects the current actual blood glucose value of the user and periodically sends the blood glucose value to the electronic module 101, for example, every 5 minutes, or every 2 minutes, or every 1 minute, or even every 30s, or other time interval, and the electronic module 101 receives and stores the blood glucose value detected by the detection module 100. Step 201, obtain the actual blood glucose value of the user at the current time; Step 202, obtain the user's historical actual blood glucose value at the previous time, and calculate the difference between the actual blood glucose values at the current time and the actual blood glucose value the previous time;

$$G_{dev}(N) = G(N) - G(N-1)$$

Where:

G(N) represents the actual blood glucose value at the current time;

G(N-1) represents the actual blood glucose value at the previous time;

$G_{dev}(N)$ represents the difference between the actual blood glucose values at the current time.

Before calculating the difference between the actual blood glucose value at the current time and the actual blood glucose value the previous time, the original continuous glucose data can also be filtered or smoothed.

Step 203, calculate the change rate of the difference between the actual blood glucose values at the current time:

$$G_{accel}(N) = \frac{(G_{dev}(N) - G_{dev}(N-1))}{Ts}$$

Where:

$G_{dev}$ (N) represents the difference between the actual blood glucose values at the current time;

$G_{dev}$ (N-1) represents the difference between the actual blood glucose values at the previous time;

$T_S$ represents the time interval between the previous time and the current time;

$G_{accel}$ (N) represents the change rate of the difference between the actual blood glucose values at the current time.

Here, the difference between the actual blood glucose values at the previous moment can be calculated by referring to step 202. The time interval Ts between the previous time and the current time can be the minimum period that detected and sent by the detection module 100 or multiple of the minimum period or, or it can be less than the minimum period that detected and sent by the detection module 100. However, in steps 202 and 203, the time interval between the current time and the previous time is the same.

Step 204, determine whether the change rate of the difference between the actual blood glucose values is positive; If yes, step 205, compare the change rate of the difference between the actual blood glucose value with the preset positive threshold; If not, step 206, compare the change rate of the difference between the actual blood glucose values with the preset negative threshold; Since the blood glucose level in the user's body will fluctuate to a certain extent without eating or exercising, setting the corresponding threshold can better determine whether the change rate of the difference in blood glucose value is caused by eating or exercising. Positive and negative thresholds can be set based on empirical values or user-defined. If the change rate of the difference between the actual blood glucose values is higher than the preset positive threshold, step 207, determined the event as a meal event, otherwise, it will not be determined as a meal event. If the change rate of the difference between the actual blood glucose values is lower than the preset negative threshold, step 208, determined the event as an exercise event, otherwise, it will not be determined as an exercise event.

During the beginning of meal or exercise, generally within 20 minutes, the change of the blood glucose value will accelerate. In order to more accurately identify the change rate of the difference between the actual blood glucose values at the current time, when the meal or exercise is detected, the detection module 100 can shorten the detection interval, for example, Ts changes from 2 minutes to 1 minute, half a minute, 20 seconds, 10 seconds, 5 seconds, or even shorter, and increase the sampling frequency to more accurately identify the meal or exercise event.

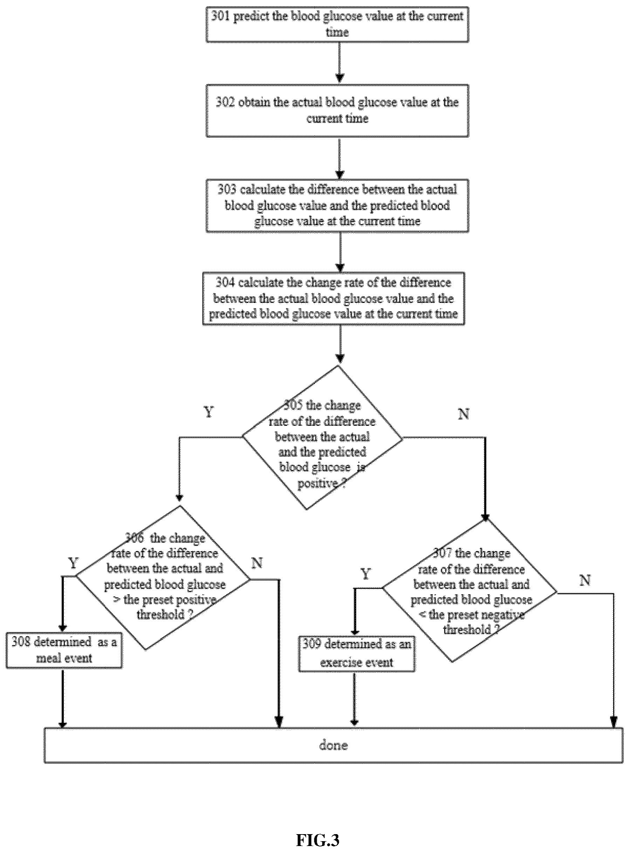

FIG.3 is a flow chart of determining the meal or exercise event based on the rate of change of the difference between the actual blood glucose value and the predicted blood glucose value according to one embodiment of the present invention.

Because blood glucose will increase or decrease rapidly during eating or exercise, predicting the blood glucose value continuously during the process, and comparing the predicted blood glucose value with the actual blood glucose value, it can be found that the difference between the predicted blood glucose value and the actual blood glucose value has an increasing trend.

Step 301, predict the blood glucose value at the current time;

In the embodiment of the invention, the blood glucose prediction algorithm is as follows:

$$G_P(N) = \sum_{k=1}^{m} (a_k * G(N-k) + b_k * CF * (I(N-k) - I_{basal}))$$

$G_p$(N) represents the predicted blood glucose value at the current time;

G(N-k) represents the actual blood glucose value at the previous k time;

$a_k$ represents a time-related coefficient;

$b_k$ represents the time decay coefficient related to the insulin consumption curve;

CF represents insulin sensitivity;

I(N-k) represents the amount of insulin infusion in the time period at time k;

$I_{basal}$ represents the basal insulin consumption of the human body in each time period.

In the embodiment of the invention, Ts represents the time period of blood glucose detection and insulin infusion, and the value of m * Ts should be greater than or equal to the IOB time, otherwise bk * CF(I(N-k) - $I_{basal}$)=0, that is, when predicting blood glucose value, only the impact of IOB in the body is considered, and the impact of IOB and $I_{basal}$ is not considered when there is no IOB in the body, and the impact of insulin sensitivity is also considered to make the predicted blood glucose value more accurate.

In another embodiment of the invention, the predicted blood glucose value is at least calculated based on the actual blood glucose value at the previous time and its first and second derivative relative to time.

Blood glucose change rate $E_v$ can be calculated from the front and rear two times, or from the linear regression of multiple times in a period of time. Specifically, when the change rate of the front and rear two moments is used, the calculation formula is:

$$E_v = \mathrm{d}G_t/\mathrm{dt} = (G_t - G_{t-1})/\mathrm{Ts}$$

Where:

$E_v$ represents the blood glucose change rate at the current time;

$G_t$ represents the blood glucose value at the current time;

$G_{t-1}$ represents the blood glucose value at the previous time;

Ts represents the time interval between the current time and the previous time.

When the blood glucose value at three points is used to calculate the change rate, the calculation formula is:

$$E_v = \mathrm{d}G_t/\mathrm{dt} = (3G_t - 4G_{t-1} + G_{t-2})/2\mathrm{Ts}$$

Where:

$G_t$ represents the blood glucose value at the current time;

$G_{t-1}$ represents the blood glucose value at the previous time;

$G_{t-2}$ represents the blood glucose value at the last previous time;

Ts represents the time interval between the current time and the previous time;

$E_v$ represents the blood glucose change rate at the current time.

Before the calculation of blood glucose change rate $E_v$, the original continuous glucose data can also be filtered or smoothed. The threshold value can be set to 1.8mg/mL-3mg/mL or personalized.

In other embodiments of the invention, the blood glucose change rate $E_v$ can also be obtained by using the weighted average method:

$$\bar{E}_v = \frac{\sum_{i=1}^{t} E_{vi}}{t-1}$$

Where:
$E_{vi}$ represents the blood glucose change rate at each detection point in a time period before the current time.

The blood glucose change rate $E_v$ obtained by weighted average method is smoother, as it eliminates the interference of some singular points, and improves the accuracy of the algorithm.

The blood glucose change rate is the first derivative of blood glucose value relative to time, which can be used to calculate the predicted blood glucose value:

$$G_{pt} = E_{v-1} * Ts + G_{t-1}$$

Where:

$G_{pt}$ represents the predicted blood glucose value at the current time;

Ts represents the time interval between the current time and the previous time;

$G_{t-1}$ represents the actual blood glucose value at the previous time;

$E_{v-1}$ represents the blood glucose change rate at the previous time.

The time interval Ts can be set as the detection period of the detection module 100, such as the CGM, Ts is 2 minutes. Ts can also be set more frequent than the detection period, such as 1 minute, or 30 seconds, and the CGM can also be set as the continuous detection mode.

Although blood glucose change rate $E_v$ can be used to predict the blood glucose value, but when it is at the turning point of the actual blood glucose curve, the above algorithm may be inaccurate. Therefore, the higher derivative of blood glucose value relative to time can also be introduced to predict blood glucose value, which can improve the accuracy of the algorithm.

In the preferred embodiment of the invention, the second derivative of blood glucose value relative to time is introduced, that is, the blood glucose change acceleration $E_a$ is used to predict the blood glucose value. Even when the predicted the blood glucose value is at the turning point of the actual blood glucose value, it can be accurately estimated.

In some embodiments of the invention, the calculation formula of the blood glucose change acceleration $E_a$ is:

$$E_a = \frac{E_{v0} - E_{v1}}{Ts}$$

Where:

$E_{v0}$ represents the blood glucose change rate at the current time;

$E_{v1}$ represents the blood glucose change rate at the previous time;

Ts represents the time interval between the current time and the previous time

$E_a$ represents the blood glucose change acceleration at the current time.

The calculation formula for predicting blood glucose value based on the blood glucose change acceleration $E_a$ is:

$$G_{pt} = \frac{E_a}{2} * Ts^2 + E_v * Ts + G_{t-1}$$

Where:

$G_{pt}$ represents the predicted blood glucose value at the current time;

Ts represents the time interval between the current time and the previous time

$G_{t-1}$ represents the actual blood glucose value at the previous time;

$E_v$ represents the blood glucose change rate at the current time;

$E_a$ represents the blood glucose change acceleration at the current time.

In other embodiments of the invention, the blood glucose change acceleration can also be obtained by using the weighted average method:

$$\bar{E}_a = \frac{\sum_{i=1}^{t} E_{ai}}{t-1}$$

Where:

$\bar{E}_a$ represents the average value of the blood glucose change acceleration.

$E_{ai}$ represents the blood glucose change acceleration at each detection point in a time period before the current moment.

The blood glucose change acceleration $E_a$ obtained by weighted average method is smoother, as it eliminates the interference of some singular points, and improves the accuracy of the algorithm.
In other embodiments of the invention, smooth algorithms such as quadratic curve fitting can also be used to calculate the blood glucose change rate and/or the blood glucose change acceleration:

$$\begin{pmatrix} n & \sum_{i=0}^{t} T_i & \sum_{i=0}^{t} T_i^2 \\ \sum_{i=0}^{t} T_i & \sum_{i=0}^{t} T_i^2 & \sum_{i=0}^{t} T_i^3 \\ \sum_{i=0}^{t} T_i^2 & \sum_{i=0}^{t} T_i^3 & \sum_{i=0}^{t} T_i^4 \end{pmatrix} \begin{pmatrix} C \\ E_v \\ E_a \end{pmatrix} = \begin{pmatrix} \sum_{i=0}^{t} G_{Ti} \\ \sum_{i=0}^{t} T_i G_{Ti} \\ \sum_{i=0}^{t} T_i^2 G_{Ti} \end{pmatrix}$$

Where:

$T_i$ represents the sampling time;

$G_{Ti}$ represents the blood glucose value detected at the sampling point;

C represents the curve constant;

$E_v$ represents the blood glucose change rate;

$E_a$ represents the blood glucose change acceleration.

Before using the quadratic curve fitting, it is necessary to sample the blood glucose value in the past time period to obtain a series of combinations $(G_{Ti}, T_i)$ of the blood glucose value and the sampling time. In the embodiment of the invention, at least three groups should be sampled to complete the quadratic curve fitting. In the preferred embodiment of the invention, three blood glucose values in the past time period are taken as the sampling points. In other embodiments of the invention, statistics can also be used to eliminate the outliers of sampling points and then perform quadratic curve fitting, which can eliminate the influence of singular points to the maximum extent, make the simulated curve smoother, and improve the accuracy of the algorithm.
In other embodiments of the invention, other curve fitting algorithms can also be used to calculate the blood glucose change rate $E_v$ and/or blood glucose change acceleration $E_a$, such as higher order curve fitting, which will not be repeated here.
Step 302, obtain the actual blood glucose value at the current time; Step 303, calculate the difference between the actual blood glucose value and the predicted blood glucose value at the current time;

$$G_{pdev}(N) = G(N) - G_p(N)$$

Where:

$G_{pdev}(N)$ represents the difference between the actual blood glucose value and the predicted blood glucose value at the current time;

$G(N)$ represents the actual blood glucose value at the current time;

$G_p(N)$ represents the predicted blood glucose value at the current time.

When eating, the $G_{pd,v}(N)$ will be greater than zero and will become larger. When exercising, $G_{pdev}(N)$ will be less than zero and will become smaller. Therefore, in step 303, it can be preliminarily determined whether the user eats or exercises.

Further, it further includes step 304, calculate the change rate of the difference between the actual blood glucose value and the predicted blood glucose value at the current time;

$$G_{paccel}(N) = \frac{(G_{pdev}(N) - G_{pdev}(N-1))}{Ts}$$

Where:

$G_{pdev}(N)$ represents the difference between the actual blood glucose value and the predicted blood glucose value at the current time;

$G_{pdev}(N-1)$ represents the difference between the actual blood glucose value and the predicted blood glucose value at the previous time;

$Ts$ represents the time interval between the previous time and the current time;

$G_{paccel}(N)$ represents the change rate of the difference between the actual blood glucose value and the predicted blood glucose value at the current time.

Here, the time interval Ts between the previous time and the current time can be the minimum period that detected and sent by the detection module 100 or multiple of the minimum period or, or it can be less than the minimum period that detected and sent by the detection module 100. The calculation method of the difference between the actual blood glucose value and the predicted blood glucose value at the previous moment is consistent with step 303.

Step 305, determine whether the change rate of the difference between the actual blood glucose value and the predicted blood glucose value at the current time is positive; If yes, step 306, compare the change rate of the difference between the actual blood glucose value and the predicted blood glucose value at the current time with the preset positive threshold; If not, step 307, compares the change rate of the difference between the actual blood glucose value and the predicted blood glucose value at the current time with the preset negative threshold; Since the blood glucose level in the user's body will fluctuate to a certain extent without eating or exercising, setting the corresponding threshold can better determine whether the change rate of the difference in blood glucose value is caused by eating or exercising. Positive and negative thresholds can be set based on empirical values or user-defined. If the change rate of the difference between the actual blood glucose value and the predicted blood glucose value at the current time is higher than the preset positive threshold, step 308, the event will be determined as a meal event, otherwise, it will not be determined as a meal event. If the change rate of the difference between the actual blood glucose value and the predicted blood glucose value at the current time is lower than the preset negative threshold, step 309, the event will be determined as an exercise event, otherwise, it will not be determined as an exercise event.

In other embodiments of the invention, the meal event or the exercise event can also be determined by combining the judgment results of the change rate of the difference between the actual blood glucose values and the change rate of the difference between the actual blood glucose value and the predicted blood glucose value at the current time, such as, if the judgment result of the change rate of the difference between the actual blood glucose values and the judgment result of the change rate of the difference between the actual blood glucose value and the predicted blood glucose value at the current time is consistent, the event can be determined as a meal or exercise event, otherwise it can not be determined as a meal or exercise event.

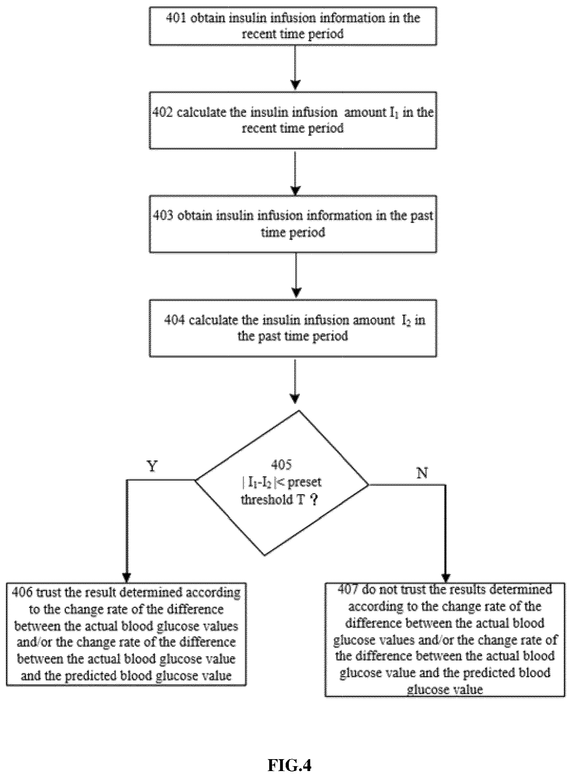

FIG.4 is a flow chart of determining the meal or exercise event by combining the insulin infusion information according to one embodiment of the present invention.

Because the change of blood glucose may be caused by abnormal insulin infusion, the recent insulin infusion information can be referred to determine the meal or exercise events.

Step 401, obtain insulin infusion information in the recent time period; Step 402, calculate the amount of insulin infusion $I_1$ in the recent time period; Step 403, obtain insulin infusion information in the past time period; Step 404, calculate the amount of insulin infusion $I_2$ in the past time period; here, the latest time period is m * Ts. As mentioned earlier, the value of m * Ts should be greater than or equal to the IOB time, while the last time period can be the m * Ts time adjacent to the latest time period; Step 405, compare the insulin infusion amount $I_1$ in the recent time period with the insulin infusion amount $I_2$ in the past time period. If the absolute value of the difference between the two is not greater than the preset threshold T, then step 406, trust the result determined according to the change rate of the difference between the actual blood glucose values and/or the change rate of the difference between the actual blood glucose value and the predicted blood glucose value at the current time, otherwise, step 407, do not trust the results determined according to the change rate of the difference between the actual blood glucose values and/or the change rate of the difference between the actual blood glucose value and the predicted blood glucose value at the current time. The change of blood glucose may be caused by the abnormal infusion of insulin. Wherein, the threshold T can be set according to the empirical value, or can be user-defined.

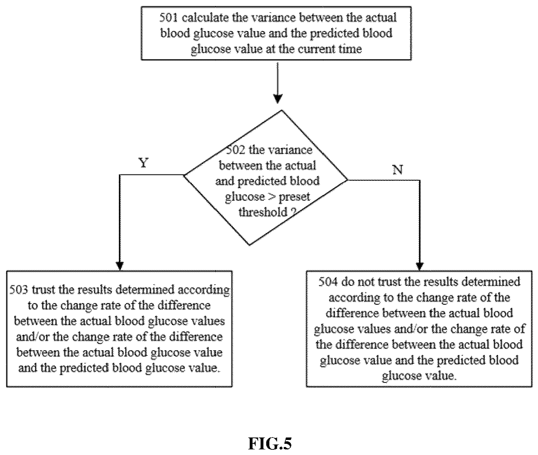

FIG.5 is a flow chart of determining the meal or exercise event based on variance of actual blood glucose value and predicted blood glucose value according to one embodiment of the present invention.

In the embodiment of the invention, eating or exercise event can also be determined by calculating the variance between the actual blood glucose value and the predicted blood glucose value at the current time.

Step 501, calculate the variance between the actual blood glucose value and the predicted blood glucose value at the current time; The calculation formula is as follows:

$$Gssr(N) = \frac{\sum_{k=1}^{m}(G(N-k)-G_p(N-k))^2}{m-1}$$

$G_{ssr}(N)$ represents the variance between the actual blood glucose value and the predicted blood glucose value at the current time;

G(N) represents the actual blood glucose value at the current time;

$G_p(N)$ represents the predicted blood glucose value at the current time.

The calculation formula of $G_p(N)$ is consistent with that in step 301.

Step 502, determine whether the variance between the actual blood glucose value and the predicted blood glucose value at the current time is greater than the preset threshold value. If the variance is greater than the threshold value, step 503, trust the results determined according to the change rate of the difference between the actual blood glucose values and/or the change rate of the difference between the actual blood glucose value and the predicted blood glucose value at the current time, if not, step 504, do not trust the results determined according to the change rate of the difference between the actual blood glucose values and/or the change rate of the difference between the actual blood glucose value and the predicted blood glucose value at the current time.

In other embodiments of the present invention, before determining the eating or exercise event by calculating the variance between the actual blood glucose value and the predicted blood glucose value at the current time it is also possible that determine the amount of insulin infusion $I_1$ in the recent time period and the amount of insulin infusion $I_2$ in the past time period first. If the absolute value of the difference between $I_1$ and $I_2$ is less than the preset threshold T, trust the results determined according to the variance between the actual blood glucose value and the predicted blood glucose value at the current time. Otherwise, do not trust the results determined according to the variance between the actual blood glucose value and the predicted blood glucose value at the current time. Here, the threshold T can be set according to the empirical value, or can be user-defined.

The motion sensor may be provided in the detection module 100, the electronic module 101, or the infusion module 102. Preferably, in the embodiment of the invention, the motion sensor is arranged in the electronic module 101.

It should be noted that, in the embodiment of the invention, the number of motion sensors and the setting positions of multiple motion sensors are not limited, as long as it can meet the conditions that the user activity can be sensed by the motion sensors.

Motion sensors include three-axis acceleration sensors or gyroscopes. Three-axis acceleration sensors or gyroscopes can more accurately sense the activity intensity, activity mode or body posture. Preferably, in the embodiment of the invention, the motion sensor is the combination of a three-axis acceleration sensor and a gyroscope.

In summary, the present invention discloses an automatic detection method based on the change rate of the difference between the actual blood glucose values, including: obtaining an actual blood glucose value of the user at the current time; obtaining a historical actual blood glucose value of the user at the previous time, and calculate a difference between the actual blood glucose values at the current time and at the previous time; calculating a change rate of the difference between the actual blood glucose values at the current time; and comparing the change rate of the difference between the actual blood glucose value at the current time with a preset threshold, and determining an event type according to the comparing result. According to the determined event type, the artificial pancreas can automatically adjust the corresponding infusion strategy to achieve closed-loop control of the artificial pancreas.

While the invention has been described in detail with reference to the specific embodiments of the present invention, it should be understood that it will be appreciated by those skilled in the art that the above embodiments may be modified without departing from the scope and spirit of the invention. The scope of the invention is defined by the appended claims.

## Claims

1. An automatic detection method based on the change rate of the difference between the actual blood glucose values, comprising:

   obtaining an actual blood glucose value of the user at the current time;
   obtaining a historical actual blood glucose value of the user at the previous time, and calculate a difference between the actual blood glucose values at the current time and at the previous time;
   calculating a change rate of the difference between the actual blood glucose values at the current time; and comparing the change rate of the difference between the actual blood glucose values at the current time with a preset threshold, and determining an event type according to the comparing result.

2. The automatic detection method based on the change rate of the difference between the actual blood glucose values of claim 1, wherein,
   when the change rate of the difference between the actual blood glucose values at the current time is positive and greater than a preset positive threshold, the event is determined as eating.

3. The automatic detection method based on the change rate of the difference between the actual blood glucose values of claim 1, wherein,
   when the change rate of the difference between the actual blood glucose values at the current time is negative and less than a preset negative threshold, the event is determined as exercise.

4. The automatic detection method based on the change rate of the difference between the actual blood glucose values of claim 1, wherein,
   when the event type is initially determined, the time interval between the current moment and the previous moment is gradually shortened.

5. The automatic detection method based on the change rate of the difference between the actual blood glucose values of claim 1, wherein,
   further determine the event type in combination with other judgment results.

6. The automatic detection method based on the change rate of the difference between the actual blood glucose values of claim 5, wherein,
   the other judgment results are based on the change rate of the difference between the actual blood glucose value and the predicted blood glucose value at the current time.

7. The automatic detection method based on the change rate of the difference between the actual blood glucose values of claim 6, wherein,
   when the judgment result obtained from the change rate of the difference between the actual blood glucose value and the predicted blood glucose value at the current time is consistent with the judgment result obtained from the change rate of the difference between the actual blood glucose values, the event type is determined.

8. the automatic detection method based on the change rate of the difference between the actual blood glucose values of claim 7, wherein,
the influence of insulin on board and insulin sensitivity are considered in the prediction algorithm for predicting blood glucose value.

9. The automatic detection method based on the change rate of the difference between the actual blood glucose values of claim 7, wherein,
the predicted blood glucose value is at least calculated based on the actual blood glucose value at the previous moment and the first or second derivative of the actual blood glucose value at the previous moment relative to time.

10. The automatic detection method based on the change rate of the difference between the actual blood glucose values of any one claim 1-9, wherein,
the other judgment results further include the results determined according to the insulin infusion information.

11. The automatic detection method based on the change rate of the difference between the actual blood glucose values of claim 10, wherein,
the method for determining the result according to the insulin infusion information includes:

obtaining an insulin infusion information in a recent time period and calculating an insulin infusion amount $I_1$;
obtaining an insulin infusion information in a past time period of time and calculating an insulin infusion amount $I_2$;
calculating the absolute value of the difference between $I_1$ and $I_2$;
comparing an absolute value of the difference with a preset threshold value, if the absolute value of the difference is not greater than the preset threshold value, trust the result determined by the change rate of the difference between the actual blood glucose values and/or the change rate of the difference between the actual blood glucose value and the predicted blood glucose value at the current time.

12. The automatic detection method based on the change rate of the difference between the actual blood glucose values of claim 11 wherein,
the past time period is adjacent to the latest time period.

13. The automatic detection method based on the change rate of the difference between the actual blood glucose values of any one claim 1-9, wherein,
the other judgment results further include the results determined according to a variance between the actual blood glucose value and the predicted blood glucose value at the current time.

14. The automatic detection method based on the change rate of the difference between the actual blood glucose values of claim 13, wherein,
the method for determining the result based on the variance between the actual blood glucose value and the predicted blood glucose value at the current time includes: comparing the variance with a preset threshold, if the variance is greater than the threshold, trust the result determined based on the change rate of the difference between the actual blood glucose values and/or the change rate of the difference between the actual blood glucose value and the predicted blood glucose value.

15. The automatic detection method based on the change rate of the difference between the actual blood glucose values of claim 13, wherein,
the other judgment results further include the results determined based on insulin infusion information.

16. The automatic detection method based on the change rate of the difference between the actual blood glucose values of claim 15, wherein,
when the absolute value of the difference between the insulin infusion amount $I_1$ in the recent time period and the insulin infusion amount $I_2$ in the past time period is less than a preset threshold, trust the result determined according to the variance between the actual blood glucose value and the predicted blood glucose value at the current time.

17. A closed-loop artificial pancreas, comprising,

a detection module, for continuously detecting a real-time blood glucose value of a user;
an infusion module, for performing currently needed insulin infusion to the user according to an insulin infusion instruction; and

an electronic module, for controlling the operation of the detection module and the infusion module, including a memory and a processor, wherein,

the processor automatically determines an event type according to a change rate of the difference between actual blood glucose values calculated based on the actual blood glucose values detected by the detection module.

18. The closed-loop artificial pancreas of claim 17, wherein,
the method for automatically determining the type of event includes:

obtaining an actual blood glucose value of the user at the current time;
obtaining a historical actual blood glucose value of the user at the previous time, and calculate a difference between the actual blood glucose values at the current time and at the previous time;
calculating a change rate of the difference between the actual blood glucose values at the current time; and
comparing the change rate of the difference between the actual blood glucose values at the current time with a preset threshold, and determining event type according to the comparing result.

19. The closed-loop artificial pancreas of claim 22, wherein,
when the change rate of the difference between the actual blood glucose values at the current time is positive and greater than a preset positive threshold, the event is determined as eating; when the change rate of the difference between the actual blood glucose values at the current time is negative and less than a preset negative threshold, the event is determined as exercise.

20. The closed-loop artificial pancreas of claim 19, wherein,
the method of automatically determining the event type is further combined with other judgment results to jointly determine the type of event.

21. The closed-loop artificial pancreas of claim 20, wherein,
the other judgment results are based on the change rate of the difference between the actual blood glucose value and the predicted blood glucose value at the current time.

22. The closed-loop artificial pancreas of claim 21, wherein,
the other judgment results further include the results determined according to the insulin infusion information.

23. The closed-loop artificial pancreas of claim 22, wherein,
the method for determining the result according to the insulin infusion information includes:

obtaining an insulin infusion information in the recent time period and calculating the insulin infusion amount $I_1$;
obtaining an insulin infusion information in the past time period of time and calculating the insulin infusion amount $I_2$;
calculating the absolute value of the difference between $I_1$ and $I_2$;
comparing an absolute value of the difference with a preset threshold value, if the absolute value of the difference is not greater than the preset threshold value, trust the result determined by the change rate of the difference between the actual blood glucose values and/or the change rate of the difference between the actual blood glucose value and the predicted blood glucose value at the current time.

24. The closed-loop artificial pancreas of claim 21, wherein,
the other judgment results further include the results determined according to a variance between the actual blood glucose value and the predicted blood glucose value at the current time.

25. The closed-loop artificial pancreas of claim 24, wherein,
the method for determining the result based on the variance between the actual blood glucose value and the predicted blood glucose value at the current time includes: comparing the variance with a preset threshold, if the variance is greater than the threshold, trust the result determined based on the change rate of the difference between the actual blood glucose values and/or the change rate of the difference between the actual blood glucose value and the predicted blood glucose value.

26. The closed-loop artificial pancreas of claim 24, wherein,
the results determined according to the variance between the actual blood glucose value and the predicted blood glucose value at the current time further include the results determined according to the insulin infusion information.

27. The closed-loop artificial pancreas of claim 17, wherein,
    further includes a motion sensor, which is a combination of a three-axis acceleration sensor and a gyroscope.

28. The closed-loop artificial pancreas of claim 27, wherein,
    the detection module, the infusion module and the electronic module are at least connected or integrated to form a whole part.

29. The closed-loop artificial pancreas of claim 27, wherein,
    the detection module, the infusion module and the electronic module are arranged in different parts, respectively.

**FIG.1**

201 obtain the actual blood glucose value of the
user at the current time

202 obtain the user's historical actual blood
glucose value at the previous time

203 calculate the change rate of the difference
between the actual blood glucose values at the
current time

204 the change rate
of the difference
between the actual
blood glucose values
is positive ?

Y          N

205 the change rate of
the difference between
the actual blood glucose
value > preset positive
threshold ?

Y          N

207 determined as a
meal event

206 the change rate of
the difference between
the actual blood glucose
values < preset negative
threshold ?

Y          N

208 determined as an
exercise event

done

**FIG.2**

```
┌─────────────────────────────────────┐
│ 301 predict the blood glucose value  │
│         at the current time          │
└─────────────────────────────────────┘
                  │
                  ▼
┌─────────────────────────────────────┐
│ 302 obtain the actual blood glucose  │
│      value at the current time       │
└─────────────────────────────────────┘
                  │
                  ▼
┌─────────────────────────────────────┐
│ 303 calculate the difference between │
│  the actual blood glucose value and  │
│   the predicted blood glucose value  │
│         at the current time          │
└─────────────────────────────────────┘
                  │
                  ▼
┌─────────────────────────────────────┐
│ 304 calculate the change rate of the │
│ difference between the actual blood  │
│ glucose value and the predicted blood│
│  glucose value at the current time   │
└─────────────────────────────────────┘
                  │
                  ▼
```

305 the change rate of the difference between the actual and the predicted blood glucose is positive?

Y — 306 the change rate of the difference between the actual and predicted blood glucose > the preset positive threshold ?

N

Y → 308 determined as a meal event

N → 307 the change rate of the difference between the actual and predicted blood glucose < the preset negative threshold ?

Y → 309 determined as an exercise event

N

done

**FIG.3**

```
┌─────────────────────────────────────┐
│ 401 obtain insulin infusion information in the │
│          recent time period          │
└─────────────────────────────────────┘
                    │
                    ▼
┌─────────────────────────────────────┐
│ 402 calculate the insulin infusion amount I₁ in the │
│          recent time period          │
└─────────────────────────────────────┘
                    │
                    ▼
┌─────────────────────────────────────┐
│ 403 obtain insulin infusion information in the past │
│             time period             │
└─────────────────────────────────────┘
                    │
                    ▼
┌─────────────────────────────────────┐
│ 404 calculate the insulin infusion amount I₂ in │
│          the past time period         │
└─────────────────────────────────────┘
                    │
                    ▼
```

$$405$$
$$|I_1 - I_2| < \text{preset threshold } T\ ?$$

Y ◄─────────────────────────────► N

```
┌─────────────────────────────────┐   ┌─────────────────────────────────┐
│ 406 trust the result determined according │   │ 407 do not trust the results determined │
│   to the change rate of the difference   │   │   according to the change rate of the   │
│ between the actual blood glucose values  │   │   difference between the actual blood   │
│ and/or the change rate of the difference │   │ glucose values and/or the change rate of │
│ between the actual blood glucose value   │   │ the difference between the actual blood │
│  and the predicted blood glucose value   │   │  glucose value and the predicted blood  │
│                                         │   │            glucose value            │
└─────────────────────────────────┘   └─────────────────────────────────┘
```

**FIG.4**

501 calculate the variance between the actual
blood glucose value and the predicted blood
glucose value at the current time

502 the variance
between the actual
and predicted blood
glucose > preset
threshold 2

Y

N

503 trust the results determined according
to the change rate of the difference
between the actual blood glucose values
and/or the change rate of the difference
between the actual blood glucose value
and the predicted blood glucose value.

504 do not trust the results determined
according to the change rate of the
difference between the actual blood
glucose values and/or the change rate of
the difference between the actual blood
glucose value and the predicted blood
glucose value.

**FIG.5**

## INTERNATIONAL SEARCH REPORT

| | International application No. |
|---|---|
| | **PCT/CN2023/078582** |

| **A.** | **CLASSIFICATION OF SUBJECT MATTER** |
|---|---|

G16H50/30(2018.01)i; A61M5/172(2006.01)i

According to International Patent Classification (IPC) or to both national classification and IPC

| **B.** | **FIELDS SEARCHED** |
|---|---|

Minimum documentation searched (classification system followed by classification symbols)

G16H,A61M

Documentation searched other than minimum documentation to the extent that such documents are included in the fields searched

Electronic data base consulted during the international search (name of data base and, where practicable, search terms used)

CNABS, CNTXT, ENTXTC, VEN: 变化, 变化率, 餐食, 差, 检测, 进餐, 进食, 人工胰腺, 膳食, 上海移宇, 事件, 速率, 血糖, 饮食, 运动, blood glucose, insulin, pancreas, eating, drop, rate

| **C.** | **DOCUMENTS CONSIDERED TO BE RELEVANT** | |
|---|---|---|

| Category* | Citation of document, with indication, where appropriate, of the relevant passages | Relevant to claim No. |
|---|---|---|
| Y | US 2015217053 A1 (ASEKO, INC.) 06 August 2015 (2015-08-06)<br>claims 1-30, and description, paragraphs 0057-0136 | 1-29 |
| Y | CN 102500013 A (BEIJING UNIVERSITY OF CHEMICAL TECHNOLOGY) 20 June 2012 (2012-06-20)<br>claims 1-9 | 1-29 |
| Y | CN 109999270 A (BEIJING INSTITUTE OF TECHNOLOGY et al.) 12 July 2019 (2019-07-12)<br>claims 1-4, description, paragraphs 0003 and 0043-0085, and figure 1 | 17-29 |
| A | CN 115568821 A (SUZHOU HEERKANG MEDICAL TECHNOLOGY CO., LTD.) 06 January 2023 (2023-01-06)<br>entire document | 1-29 |
| A | CN 115064261 A (THE PEOPLE'S HOSPITAL OF LONGHUA, SHENZHEN et al.) 16 September 2022 (2022-09-16)<br>entire document | 1-29 |

| ☐ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **14 June 2023** | **21 June 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/078582**

| Patent document cited in search report | | | Publication date (day/month/year) | Patent family member(s) | | | Publication date (day/month/year) |
|---|---|---|---|---|---|---|---|
| US | 2015217053 | A1 | 06 August 2015 | US | 2022248989 | A1 | 11 August 2022 |
| | | | | JP | 2019213866 | A | 19 December 2019 |
| | | | | EP | 3046599 | A1 | 27 July 2016 |
| | | | | US | 2018122505 | A1 | 03 May 2018 |
| | | | | US | 2017076067 | A1 | 16 March 2017 |
| | | | | US | 2017007761 | A1 | 12 January 2017 |
| | | | | HK | 1222357 | A1 | 30 June 2017 |
| | | | | WO | 2015116371 | A1 | 06 August 2015 |
| | | | | US | 2023087860 | A1 | 23 March 2023 |
| | | | | JP | 2017505695 | A | 23 February 2017 |
| | | | | AU | 2015211352 | A1 | 05 May 2016 |
| | | | | US | 2017281098 | A1 | 05 October 2017 |
| | | | | EP | 3926638 | A1 | 22 December 2021 |
| | | | | IL | 246936 | A0 | 02 November 2016 |
| | | | | CA | 2926761 | A1 | 06 August 2015 |
| CN | 102500013 | A | 20 June 2012 | None | | | |
| CN | 109999270 | A | 12 July 2019 | None | | | |
| CN | 115568821 | A | 06 January 2023 | None | | | |
| CN | 115064261 | A | 16 September 2022 | None | | | |

Form PCT/ISA/210 (patent family annex) (July 2022)